# EUROPEAN PATENT APPLICATION

(11) **EP 4 775 991 A1**
(43) Date of publication of application: **15.07.2026**
(21) Application number: 25382016.1
(22) Date of filing: 14.01.2025
(51) Int. Cl.: G01N 33/574, C12Q 1/6886

(54) **IN VITRO METHOD FOR THE PROGNOSIS OF PATIENTS SUFFERING FROM BREAST CANCER AND/OR FOR PREDICTING METASTASIS IN PATIENTS SUFFERING FROM BREAST CANCER**

(71) Applicant: Fundación Pública Galega Instituto de Investigación Sanitaria de Santiago de Compostela, 15706 Santiago de Compostela A Coruña (ES); Servizo Galego De Saúde, 15703 Santiago de Compostela, A Coruna (ES); Universidade de Santiago de Compostela (USC), 15872 Santiago de Compostela A Coruña (ES)
(72) Inventor: FERNÁNDEZ SANTIAGO, Cristóbal, 15706 Santiago de Compostela (ES); PIÑEIRO CID, Roberto, 15706 Santiago de Compostela (ES); LÓPEZ LÓPEZ, Rafael, 15706 Santiago de Compostela (ES)
(74) Representative: Hoffmann Eitle

(57) **Abstract**

The present invention refers to the medical field. Particularly, it refers to an *in vitro* method for the prognosis of patients suffering from breast cancer and/or for predicting metastasis in patients suffering from breast cancer.

## Description

### FIELD OF THE INVENTION

The present invention refers to the medical field. Particularly, it refers to an *in vitro* method for the prognosis of patients suffering from breast cancer and/or for predicting metastasis in patients suffering from breast cancer.

### STATE OF THE ART

Breast cancer is a major public health problem and one of the most frequent malignant neoplasms among women worldwide. Despite substantial advances in early detection, treatment, and overall survival rates, breast cancer remains one of the leading causes of cancer deaths each year. The biological heterogeneity of breast cancer encompasses a range of molecular subtypes and clinical manifestations, further frustrating efforts to identify uniform diagnostic and prognostic tests. In less advanced stages of the disease, the prognosis is typically not quite so dire; hence, there is a need for more specific predictors of metastasis recurrence.

Basically, breast cancer can be complex by being, in principle, divided into several molecular subtypes, such as luminal A, luminal B, HER2-enriched, and triple-negative breast cancers. Besides the heterogeneity of the biological features of these subtypes, a very different response to therapy may be obtained for each subtype, which makes clinical decision-making even more complicated. Accordingly, in light of the disease's heterogeneous nature, accurate risk stratification methods have been made an important area of research.

Over the past decade multigene expression signatures have been used to predict recurrence and metastasis. Well-recognized examples include MammaPrint and Oncotype DX, both in widespread use now to guide treatment decisions in specific classes of breast cancer. MammaPrint is a 70-gene signature applied mainly to early-stage breast cancers where the tumor is smaller, node-negative, and hormone receptor-positive or -negative. Oncotype DX involves 21 genes and is intended for estrogen receptor-positive, HER2-negative cancers. It is particularly useful in identifying those individuals for whom chemotherapy may be of benefit in the early stage of disease. These are now indispensable tools in the clinical armamentarium, but they are licensed only for specific subgroups of patients and do not meet the needs of patients with more advanced disease or those of different molecular profiles.

Circulating tumor cells (CTCs), especially clusters of CTCs, form yet another very promising area of study in metastasis understanding regarding breast cancer. CTCs are cells that break away from the primary tumor into the bloodstream, where eventually they may form secondary tumors in distant organs. Indeed, the clusters of CTCs, which are small groups of tumor cells that travel together, have greater metastatic potential compared to single CTCs. Hence, they are an important focus necessary to understand the mechanisms of cancer spread. However, the molecular underpinnings that drive the enhanced metastatic capacity of CTC clusters remain poorly defined. Such genes that are specifically upregulated in CTC clusters may serve as good biomarkers for predicting metastatic risk and guiding treatment.

There is an unmet medical need of finding less complex and reliable strategies for the prognosis of patients suffering from breast cancer and/or for predicting metastasis in patients suffering from breast cancer. The present invention is focused on solving this problem and an innovative strategy for the prognosis of patients suffering from breast cancer and/or for predicting metastasis in patients suffering from breast cancer is herein provided.

### Description of the invention

### Brief description of the invention

The present invention refers to an *in vitro* method for the prognosis of patients suffering from breast cancer and/or for predicting metastasis in patients suffering from breast cancer.

Particularly, the inventors of the present invention identified genes consistently upregulated in CTC clusters versus single CTCs using three datasets: GSE51827, GSE67939, GSE86978. Comprehensive intra-patient comparisons showed almost 200 genes to be consistently upregulated in CTC clusters. These genes were then used in their predictive power of metastasis, using 11 primary tumor datasets with distant metastasis-free survival (DMFS) data available: GSE11121, GSE1456, GSE16446, GSE17705, GSE19615, GSE2034, GSE20685, GSE4922, GSE5327, GSE6532, GSE9195. LASSO regression analysis narrowed it down further to four key genes: PNPO, PNRC2, PPIA, and CLU. The model was further validated in two independent data sets, GSE42568 and GSE25066.

A four-gene signature was achieved compri sing the genes CLU, PNRC2, PPIA and PNPO with an AUC of 0.70 in the validation set and 0.744 in the GSE42568 dataset. In combination with the clinical variables of node status, age at diagnosis, and tumor grade, performance rose to 0.822. However, for the younger cohort of GSE25066, the overall AUC was 0.63. Despite the variability in performance, our signature is useful across a wide population of patients and does not carry the restrictive inclusion criteria found in existing, well-established tests such as MammaPrint and Oncotype DX.

In conclusion, the four-gene signature of the invention, comprising the genes CLU, PNRC2, PPIA and PNPO, provides a more easily and broadly applicable tool for metastasis prediction than MammaPrint and Oncotype DX, which apply to limited patient subgroups.

On the other hand, although the results provided in the Examples below show that the best performance was achieved when the genes CLU, PNRC2, PPIA and PNPO were combined, according to the information provided in **Table 1,** the present invention can be also carried out by using these genes individually and also combinations thereof comprising at least two or three genes.

**Table 1**

| **Ranking** | **Genes** | **ROC (AUC) Validation cohort** |
|---|---|---|
| 1 | CLU + PNRC2 + PPIA + PNPO | 0.744 |
| 2 | PNRC2 + PPIA + PNPO | 0.7068 |
| 3 | CLU + PPIA + PNPO | 0.6912 |
| 4 | CLU + PNR2 + PNPO | 0.6905 |
| 5 | PNRC2 + PNPO | 0.6823 |
| 6 | CLU + PNR2 | 0.6674 |
| 7 | CLU + PNPO | 0.66 |
| 8 | PNRC2 | 0.6577 |
| 9 | PNRC2 + PPIA | 0.6536 |
| 10 | PPIA + PNPO | 0.6146 |
| 11 | CLU | 0.61 |
| 12 | PNPO | 0.609 |
| 13 | CLU + PPIA | 0.5815 |
| 14 | PPIA | 0.5331 |
| 15 | CLU + PNR2 + PPIA | 0.5193 |

Consequently, the first embodiment of the present invention refers to an *in vitro* method for identifying biomarker signatures for the prognosis of patients suffering from breast cancer and/or for predicting metastasis in patients suffering from breast cancer which comprises: a) Measuring the level of expression of at least two genes selected from the group consisting of: PNRC2, CLU, PNPO and/or PPIA, in a biological sample obtained from the patient; b) determining a combination score value; and c) wherein if a deviation of the combination score value is identified, as compared with a pre-established reference value, this is indicative that the biomarker signature may be used for the prognosis of patients suffering from breast cancer and/or for predicting metastasis in patients suffering from breast cancer.

The second embodiment of the present invention refers to an *in vitro* method for the prognosis of patients suffering from breast cancer and/or for predicting metastasis in patients suffering from breast cancer, the method comprising measuring the level of expression of at least one gene selected from the list consisting of PNRC2, CLU, PNPO and/or PPIA, in a biological sample obtained from the patient.

The third embodiment of the present invention refers to the *in vitro* use of at least one gene selected from the list consisting of PNRC2, CLU, PNPO and/or PPIA for the prognosis of patients suffering from breast cancer and/or for predicting metastasis in patients suffering from breast cancer.

In a preferred embodiment, the present invention comprises measuring the level of expression of a signature selected from **Table 1.**

In a preferred embodiment, the present invention comprises measuring the level of expression of a signature comprising the genes PNRC2, CLU, PNPO and PPIA, in a biological sample obtained from the patient.

In a preferred embodiment, the present invention further comprises assessing the following clinical parameters: node status, age at diagnosis and/or tumor grade.

In a preferred embodiment, if a deviation of the level of expression is identified, as compared with a pre-established reference value, this is indicative of the prognosis of patients suffering from breast cancer.

In a preferred embodiment, if a deviation of the level of expression is identified, as compared with a pre-established reference value, this is indicative that the patient has poor prognosis or that the patient may suffer from metastasis. Preferably, a lower level of expression of the genes PNPO, PNRC2 and/or CLU; and/or a higher level of expression of PPIA, as compared with a pre-established reference value, is indicative of poor prognosis or that the patient may suffer from metastasis.

In a preferred embodiment, the biological sample is selected from: tissue, plasma, serum or whole blood.

Alternatively, the present invention also refers to a method for treating patients suffering from breast cancer, wherein the method comprises, as a previous step, the prognosis of patients suffering from breast cancer and/or the prediction of metastasis in patients suffering from breast cancer by using the method of the invention. In a preferred embodiment, the patient would be treated with the following treatments:
- Hormone (Endocrine) Therapy: For patients with hormone receptor-positive (HR+) metastatic breast cancer, hormone therapy is often the first-line treatment. It aims to block estrogen receptors or reduce estrogen production, slowing cancer growth. Common drugs include aromatase inhibitors, selective estrogen receptor modulators (SERMs), and selective estrogen receptor degraders (SERDs), as well as newer drugs like CDK4/6 inhibitors (e.g., abemaciclib, palbociclib, ribociclib).
- Targeted Therapy: Targeted drugs are designed for specific proteins or genes that drive cancer growth. For HER2-positive metastatic breast cancer, drugs like trastuzumab, pertuzumab, and trastuzumab deruxtecan are effective. Newer treatments target the PI3K pathway (e.g., alpelisib) and HER2-low breast cancer (e.g., trastuzumab deruxtecan). For patients with BRCA mutations, PARP inhibitors (e.g., olaparib, talazoparib) may be options.
- Chemotherapy: Chemotherapy remains a backbone treatment, especially for patients whose cancer is hormone receptor-negative or HER2-negative, or those whose cancer has stopped responding to hormone therapy. Common drugs include taxanes (e.g., paclitaxel, docetaxel), anthracyclines, and capecitabine.
- Immunotherapy: Immunotherapy, particularly immune checkpoint inhibitors, is an option for some patients, notably those with triple-negative breast cancer (TNBC). Pembrolizumab, for instance, is used in combination with chemotherapy for PD-L1-positive TNBC.
- Bone-Directed Therapy: For patients with bone metastases, treatments such as bisphosphonates (e.g., zoledronic acid) or denosumab help reduce bone complications and alleviate pain.
- Radiation Therapy and Surgery: While less common in metastatic disease, radiation can be used to alleviate pain and control symptoms in specific areas. Surgery is generally rare but may be used in special cases to remove a limited number of metastases.
- Clinical Trials: Many patients with metastatic breast cancer participate in clinical trials to access new and experimental treatments that may be more effective than standard options.

In a preferred embodiment, the present invention is a computer-implemented invention, wherein a processing unit (hardware) and a software are configured to:
- Receive the expression level values of any of the above cited biomarkers or signatures,
- Process the expression level values received for finding substantial variations or deviations, and
- Provide an output through a terminal display of the variation or deviation of the expression level, wherein the variation or deviation of the expression level is indicative of the prognosis of the patients suffering from breast cancer.

For the purpose of the present invention the following terms are defined:
- According to the present invention, a reference value can be a "pre-established reference value" or a "cut-off" value. Typically, a "threshold value" or "cut-off value" can be determined experimentally, empirically, or theoretically. This final value is used as a "threshold" or "cut-off' to determine the patient's prognosis. According to the present invention, the "pre-established threshold" value refers to a value previously determined in patients with breast cancer before the appearance of distant recurrence or metastasis. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.
- "Score value" or "risk score value": In a preferred embodiment, LASSO coefficient was used for each of the genes of the signature, that multiplied by the value of its expression level, gives rise to a risk score. When several genes are combined (for instance; four genes in the present invention), the final risk score is the sum of the risk scores of each gene included in the combination.
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of". Thus, the phrase "consisting of" indicates that the listed elements are required or mandatory, and that no other elements may be present.

### Description of the figures

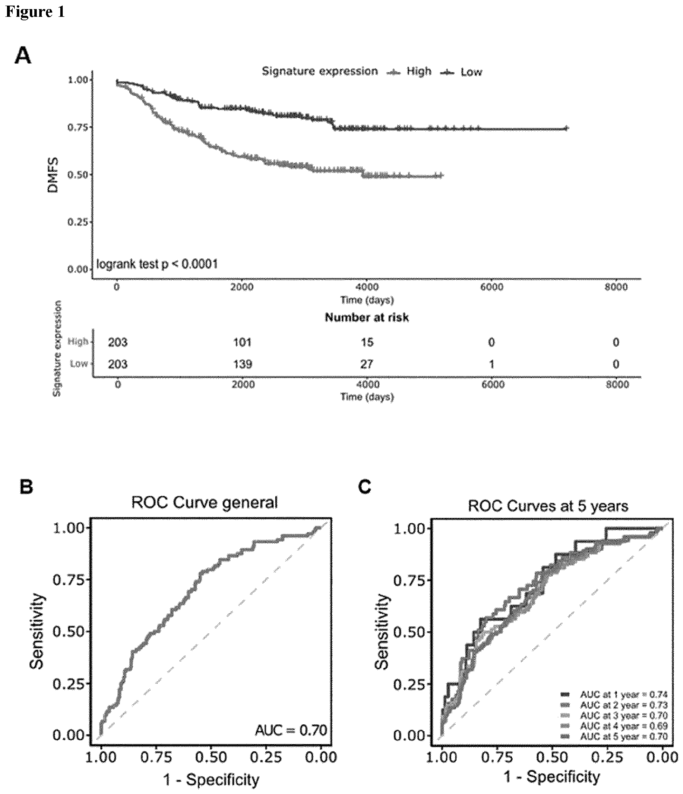

**Figure 1****.** This figure shows that the four-gene signature comprising the genes PNRC2, CLU, PNPO and PPIA was able to identify a group of high-risk patients with a significantly shorter DMFS (P < 0.0001) in the11 primary tumor datasets **(A).** Overall, the predictive value of the signature reached an area under the curve (AUC) of 0.70 in the validation set (**B**). At 1, 2, 3, 4, and 5 years, AUC values were 0.74, 0.73, 0.70, 0.69, and 0.70, respectively, showing moderate predictive power over a five-year period (**C**).

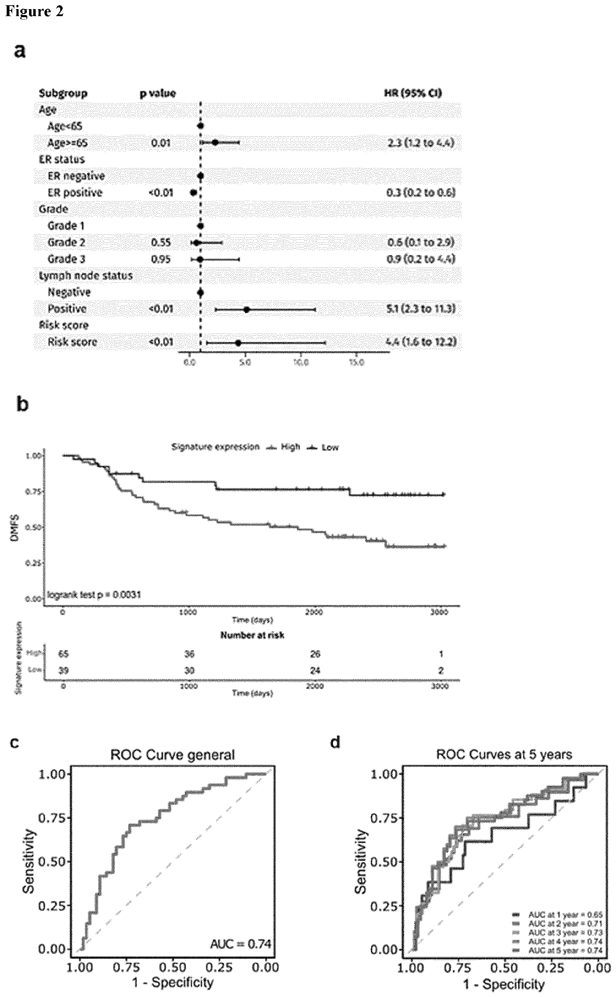

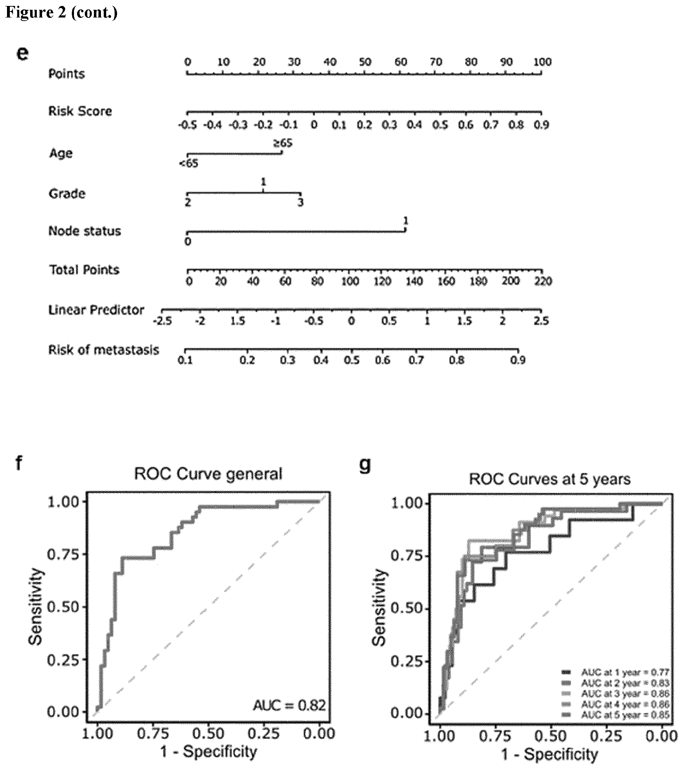

**Figure 2****.** This figure shows the GSE42568 dataset, which includes gene expression data along with comprehensive clinical variables for 108 patients, including DMFS information at per patient level **(A).** The four-gene signature maintained its prognostic value, with the high-risk group having a shorter DMFS (P = 0.0031) **(B).** Utilizing this dataset, we improved the performance of the four-gene signature, achieving an overall AUC of 0.744 and also at 4 and 5 years **(C, D).** When integrated with clinical variables of the nomogram, including node status, age, and tumor grade **(E),** the AUC further increased to 0.822 **(F).** The AUC values for selected time points are 0.77, 0.83, 0.86, 0.86, and 0.85 at 1, 2, 3, 4, and 5 years, respectively **(G),** indicating that the combination of genetic and clinical factors enhances the prediction of metastasis in a clinical context.

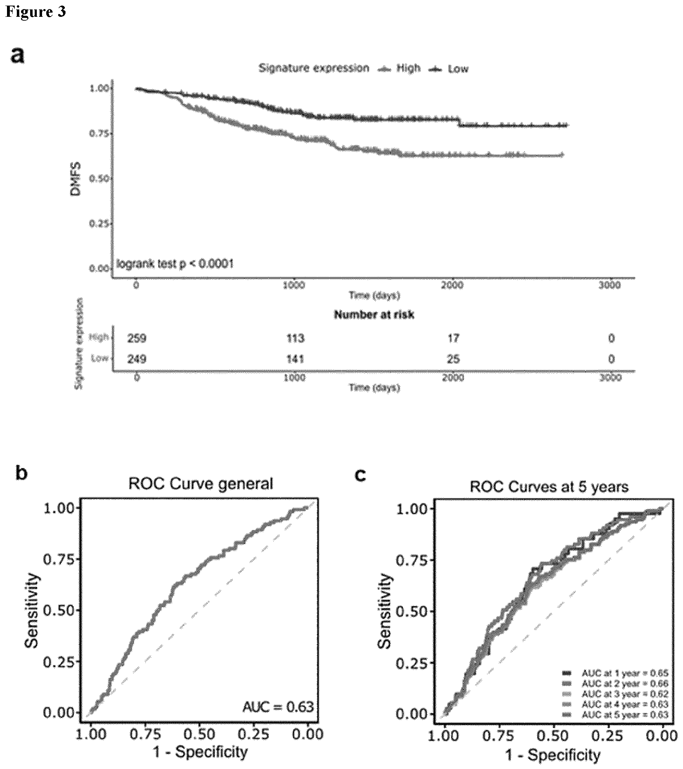

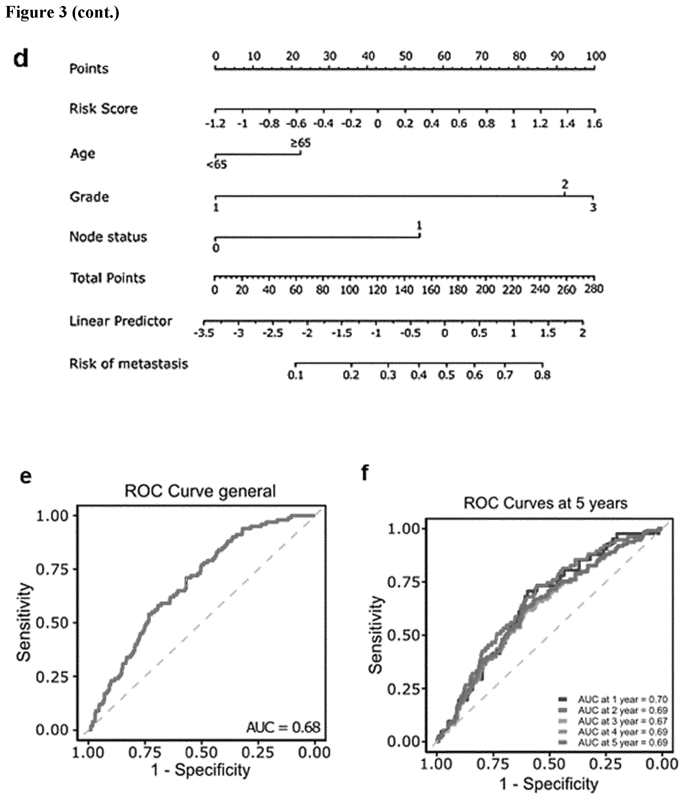

**Figure 3****.** This figure shows a validation of the signature by testing it in the GSE25066 dataset consisting of 508 patients with a younger median age of 49 years. The four-gene signature demonstrated good prognostic value in discriminating patients at risk of metastatic recurrence (P < 0.0001) **(A)** achieving an overall AUC of 0.63 and 0.66 after 2 years **(B, C).** With the inclusion of clinical variables in the nomogram **(D),** the AUC showed a modest improvement, rising to 0.6818 **(E).** The AUC values at years 1, 2, 3, 4, and 5 were 0.70, 0.69, 0.67, 0.69, and 0.69, respectively **(F),** indicating a stable predictive performance over time. The Kaplan-Meier (KM) survival curves in this dataset, and also in the validation set and GSE42568, showed significant separations of high-risk versus low-risk groups with p-values below 0.05, thus confirming the model's utility in stratifying patients into distinct risk categories.

### Detailed description of the invention

The present invention is illustrated by means of the Examples set below without the intention of limiting its scope of protection.

### Example 1. Material and methods

### Example 1.1. Transcriptomic Profiling and Differential Gene Expression Analysis

RNA-seq and microarray data were obtained from the GEO database. To preprocess and normalize the microarray data, we used the Robust Multi-array Average (RMA) through the R package affy and its rma function, which yields background correction, quantile normalization, and probe-level summarization converting the intensities into log2 expression values.

Raw count matrices of RNA-seq data were downloaded from GEO. Data were normalized and the variance stabilized using the voom function in limma. The voom method undertakes a transformation of count data for log2 counts per million (logCPM) addressing the mean-variance relationship, and thereby allowing linear modeling with limma to investigate differential expression.

For the three specific datasets (GSE51827, GSE86978 and GSE111065), we follow strictly the methodologies of their original authors. Intra-patient comparisons of CTC clusters to single CTCs were performed with the DEGseq package (version 1.56.1). Differential expression analysis (MARS method) was performed using a q-value threshold of 0.05 and fold change cut-off of 1.5, identifying genes differentially expressed between CTC clusters and single CTCs within individual patients.

### Example 1.2. Batch Effect Correction

Given that the dataset spans multiple sources and experiments following datasets consolidation, batch corrections were made using the R removeBatchEffect function (part of limma package). This step minimized technical variations between different experimental conditions, thus comparing gene expression data across datasets.

### Example 1.3. Gene Signature Selection via LASSO Regression Model

Using LASSO (Least Absolute Shrinkage and Selection Operator) regression, we then constructed a gene signature that could predict metastasis. This technique is especially useful when analysing high-dimensional data, where the number of predictors (or genes) far exceeds the number of observations or patients. LASSO is a regularization method that incorporates an L1 penalty on the absolute size of regression coefficients and implicitly performs feature selection by setting coefficients to zero for less important predictors thus aiding interpretability.

LASSO analysis was performed with the R package glmnet, and raw expression data of nearly 200 candidate genes were imported into the model. The best lambda value is chosen as the one that minimizes this error, implementing 10-fold cross-validation. This approach enabled the model to keep only genes that were the most predictive. LASSO regression prioritized 23 genes as candidates and subsequent greedy search algorithm was used to further prune the selected subset of genes. Finally, the four genes (PNPO, PNRC2, PPIA, and CLU) best reproduced the most accurate model.

### Example 1.4. Nomogram Construction

A nomogram was developed by integrating the gene signature with clinical factors (age at diagnosis, node status, and tumor grade), to improve the clinical relevance of the four-gene signature. Nomogram was constructed using rms package in R to visualize the risk score predicting metastasis by incorporating gene signature and other significant clinical factors.

Every variable, gene signature, clinical feature was given a score that when summed up together made the total risk score for each patient. And the greater the total score is revealed as higher risk to predict metastasis. The prediction model was confirmed by C-index and calibration plots to evaluate the ability of nomogram to predict occurrence probability, that is assessing the relationship in a statistical name between forecasted probabilities and observed rates (calibration).

### Example 1.5. Construction of ROC curve and Model Evaluation

To assess the predictive accuracy of the gene signature and nomogram, we calculated the area under the curve (AUC) by generating Receiver Operating Characteristic (ROC) curves using ROC function in pROC package for R both for the gene signature alone as well as combined model together with clinical variables to determine if our model is able to discriminate over time. From this, ROC curves were plotted for 1-year, 2-year, 3-year, 4-year, and 5-year distant metastasis-free survival (DMFS) to assess overall prediction performance at multiple time points.

The model AUC was considered, and the signature were compared with other clinical prediction tools like MammaPrint and Oncotype DX. ROC curve analysis allowed us to assess the tradeoff between sensitivity and specificity in predicting metastasis, with a higher AUC indicating better prediction performance.

### Example 1.6. Statistical Analysis

Statistical analyses R version 4.4.1 was further employed for all statistical analyses (differential expression analysis, LASSO regression, nomogram construction, and ROC curve construction). A significance level of 0.05 was applied as a threshold for the statistical significance in all analyses. The Kaplan-Meier survival curves were used for further validation of the model, where patients were divided into high-risk and low-risk groups by the gene signature. Survival analysis was conducted in R with the survival and survminer packages.

### Example 2. Results

### Example 2.1. Identification of a set of genes specific of CTC clusters in breast cancer

We conducted a reanalysis of three publicly available datasets: GSE51827, GSE86978, and GSE111065, which provide gene expression data (RNA-seq) for both circulating tumor cell clusters (cCTC) and single circulating tumor cells (sCTC). The GSE51827 dataset comprises data from 29 samples, including 15 pools of sCTCs and 14 cCTCs isolated from 10 breast cancer patients. The GSE86978 dataset features 55 sCTCs and 22 cCTCs from 22 patients diagnosed with metastatic ER-positive breast cancer. Additionally, GSE111065 includes 48 sCTCs and 21 cCTCs from 13 different patients.

We performed intrapatient comparisons of sCTCs and cCTCs when samples were available from the same patient. Through these comparisons, we identified approximately 200 genes that were consistently overexpressed in CTC clusters in at least 70% of the analysed cases.

### Example 2.2. Establishment of the CTC cluster-related prognostic signature in breast cancer

Given the key contribution of CTC clusters to metastasis initiation, we reasoned that the overexpression of these genes might drive a critical role in CTC cluster formation and metastatic competence. This allowed us to hypothesize that these genes may represent an important core of genes whose expression at primary tumor level may favour CTC cluster formation and disease progression.

In order to test this hypothesis, we wanted to know whether the genes upregulated in CTC clusters were able to identify patients at a greater risk of developing metastasis based on analysis of datasets that supplied gene expression data from primary tumors and information regarding distant metastasis-free survival (DMFS). For this, data were extracted from publicly available datasets in Gene Expression Omnibus (GEO), including GSE11121, GSE1456, GSE16446, GSE17705, GSE19615, GSE2034, GSE20685, GSE4922, GSE5327, GSE6532, and GSE9195. These datasets allowed us to draw data from a diverse cohort of 1,630 patients (Table 1) to assess the predictive power of the genes differentially expressed in CTC clusters versus single CTCs and their capability to predict metastasis when dysregulated at primary tumor level.

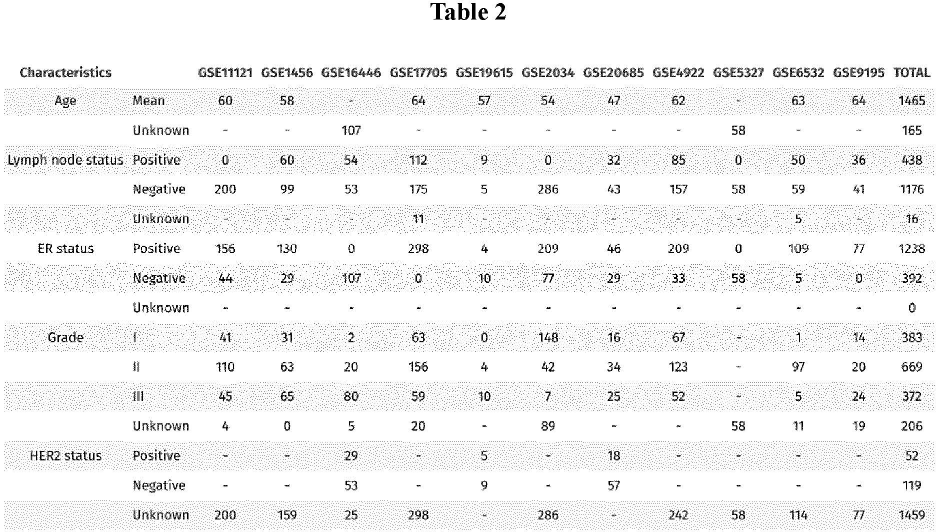

Then, we applied LASSO regression to reduce the number of genes and retain those that were most predictive. Through this process, the list went down from about 200 dysregulated genes to 23 candidates as selected by LASSO coefficients. We retained genes with coefficients greater than 0.1 or less than -0.1, as genes whose coefficients are further from zero generally have greater importance in the model. Further optimization of the gene set to select genes that best predict the outcome was done using a greedy search- known as forward selection-, a step-by-step optimization technique that selects the locally optimal solution at each stage. The approach followed herein systematically identified the subset of genes that maximized the performance of the model in predicting the metastatic onset allowing us to discriminate between risk groups. Using this process, the selected genes were pyridoxamine 5'-phosphate oxidase (PNPO), proline rich nuclear receptor coactivator 2 (PNRC2), Peptidylprolyl Isomerase A (PPIA), and Clusterin (CLU). These four genes formed the basis of our predictive model.

Once we determined this set of four genes, we again used LASSO regression to try to optimize the coefficients for each gene. Including fewer genes into our model was one way of minimizing noise while reducing the risk of overfitting and improving generalizability and performance of the model. Testing of this four-gene signature was done on the cohort of 1,630 patients that were divided into 75% training data and 25% validation. In the validation set, the four-gene signature was able to identify a group of high-risk patients with a significantly shorter DMFS (HR_{DMFS} 2.584 (95% CI 1.789-3.733), *P* < 0.0001) (**Figure 1a**). Overall, the predictive value of the signature reached an area under the curve (AUC) of 0.70 in the validation set (**Figure 1b**). At 1, 2, 3, 4, and 5 years, AUC values were 0.74, 0.73, 0.70, 0.69, and 0.70, respectively, showing moderate predictive power over a five-year period (**Figure 1c**).

### Example 2.3. Evaluation of the nomogram

While this model had robust performance, its simplicity of using only four genes offers practical advantages in terms of clinical implementation. However, this model still exhibited moderate predictive power compared to larger multigene signatures. Many predictive models are developed including additional clinical variables using a nomogram approach. Even though we had general clinical data for most of the datasets included in the initial meta-analysis, we did not have detailed per-patient information to construct a comprehensive nomogram. As a result, we were unable to incorporate additional clinical variables into the model and instead focused solely on the gene expression data.

With this limitation in mind, we identified the GSE42568 dataset, which includes gene expression data along with comprehensive clinical variables for 108 patients, including DMFS information at per patient level (**Figure 2a**). In this data set, the four-gene signature maintained its prognostic value, with the high-risk group having a shorter DMFS (HR_{DMFS} 2.753 (95% CI 1.368-5.537), *P* = 0.0031) (**Figure 2b**). Utilizing this dataset, we improved the performance of the four-gene signature, achieving an overall AUC of 0.744 and also at 4 and 5 years **(**Figure **2c****, d**). When integrated with clinical variables of the nomogram, including node status, age, and tumor grade (**Figure 2e**), the AUC further increased to 0.822 **(**Figure **2f**). The AUC values for selected time points are 0.77, 0.83, 0.86, 0.86, and 0.85 at 1, 2, 3, 4, and 5 years, respectively (**Figure 2g**), indicating that the combination of genetic and clinical factors enhances the prediction of metastasis in a clinical context.

To further validate the robustness of our signature, we tested it in the GSE25066 dataset consisting of 508 patients with a younger median age of 49 years. The four-gene signature demonstrated good prognostic value in discriminating patients at risk of metastatic recurrence (*P* < 0.0001) (**Figure 3a**) achieving an overall AUC of 0.63 and 0.66 after 2 years (**Figure 3b, c**). With the inclusion of clinical variables in the nomogram (**Figure 3d**), the AUC showed a modest improvement, rising to 0.6818 (**Figure 3e**). The AUC values at years 1, 2, 3, 4, and 5 were 0.70, 0.69, 0.67, 0.69, and 0.69, respectively (**Figure 3f**), indicating a stable predictive performance over time. The KM survival curves in this dataset, and also in the validation set and GSE42568, showed significant separations of high-risk versus low-risk groups with p-values below 0.05, thus confirming the model's utility in stratifying patients into distinct risk categories.

## Claims

1. *In vitro* method for identifying biomarker signatures for the prognosis of patients suffering from breast cancer and/or for predicting metastasis in patients suffering from breast cancer which comprises: a) Measuring the level of expression of at least two genes selected from the group consisting of: PNRC2, CLU, PNPO and/or PPIA, in a biological sample obtained from the patient; b) determining a combination score value; and c) wherein if a deviation of the combination score value is identified, as compared with a pre-established reference value, this is indicative that the biomarker signature may be used for the prognosis of patients suffering from breast cancer and/or for predicting metastasis in patients suffering from breast cancer.

2. *In vitro* method for the prognosis of patients suffering from breast cancer and/or for predicting metastasis in patients suffering from breast cancer, the method comprising measuring the level of expression of at least one gene selected from the list consisting of PNRC2, CLU, PNPO and/or PPIA, in a biological sample obtained from the patient.

3. *In vitro* use of at least one gene selected from the list consisting of PNRC2, CLU, PNPO and/or PPIA for the prognosis of patients suffering from breast cancer and/or for predicting metastasis in patients suffering from breast cancer.

4. *In vitro* method, or *in vitro* use, according to any of the claims 2 or 3, for the prognosis of patients suffering from breast cancer and/or for predicting metastasis in patients suffering from breast cancer, which comprises measuring the level of expression of a signature selected from **Table 1.**

5. *In vitro* method, or *in vitro* use, according to any of the claims 2 to 4, for the prognosis of patients suffering from breast cancer and/or for predicting metastasis in patients suffering from breast cancer, which comprises measuring the level of expression of a signature comprising the genes PNRC2, CLU, PNPO and PPIA, in a biological sample obtained from the patient.

6. *In vitro* method or *in vitro* use, according to any of the claims 2 to 5, further comprising assessing the following clinical parameters: node status, age at diagnosis and/or tumor grade.

7. *In vitro* method or *in vitro* use, according to any of the claims 2 to 6, wherein if a deviation of the level of expression is identified, as compared with a pre-established reference value, this is indicative of the prognosis of patients suffering from breast cancer.

8. *In vitro* method or *in vitro* use, according to any of the claims 2 to 7, wherein if a deviation of the level of expression is identified, as compared with a pre-established reference value, this is indicative that the patient has poor prognosis or that the patient may suffer from metastasis.

9. *In vitro* method or *in vitro* use, according to any of the claims 2 to 8, wherein if a lower level of expression of the genes PNPO, PNRC2 and/or CLU; and/or a higher level of expression of PPIAis identified, as compared with a pre-established reference value, it is indicative of poor prognosis or that the patient may suffer from metastasis.

10. *In vitro* method or *in vitro* use, according to any of the claims 2 to 9, wherein the biological sample is selected from: tissue, plasma, serum or whole blood.
